(19) Europäisches Patentamt
European Patent Office
Office européen des brevets

(11) Veröffentlichungsnummer: **0 075 144**
**A1**

(12) # EUROPÄISCHE PATENTANMELDUNG

(21) Anmeldenummer: **82107998.5**

(22) Anmeldetag: **31.08.82**

(51) Int. Cl.³: **C 07 D 233/70**, A 01 N 43/50

(30) Priorität: **12.09.81 DE 3136328**

(43) Veröffentlichungstag der Anmeldung: **30.03.83**
**Patentblatt 83/13**

(84) Benannte Vertragsstaaten: **AT BE CH DE FR GB IT LI NL**

(71) Anmelder: **BAYER AG, Zentralbereich Patente, Marken und Lizenzen, D-5090 Leverkusen 1, Bayerwerk (DE)**

(72) Erfinder: **Maurer, Fritz, Dr., Roeberstrasse 8, D-5600 Wuppertal 1 (DE)**
Erfinder: **Homeyer, Bernhard, Dr., Obere Strasse 28, D-5090 Leverkusen 3 (DE)**
Erfinder: **Hammann, Ingeborg, Dr., Belfortstrasse 9, D-5000 Köln 1 (DE)**

(54) **N,N-Dimethyl-O-imidazol-4-yl-carbaminsäureester, Verfahren zu ihrer Herstellung und ihre Verwendung als Schädlingsbekämpfungsmittel.**

(57) Die vorliegende Erfindung betrifft neue N,N-Dimethyl-O-imidazol-4-yl-carbaminsäureester der Formel (I)

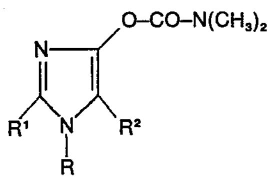

(I)

in welcher

R für gegebenenfalls substituierte Alkyl-, Alkenyl-, Alkinyl- und Cycloalkylreste steht,

$R^1$ für Wasserstoff oder für gegebenenfalls substituierte Alkyl-, Alkenyl-, Alkinyl- und Cycloalkyl-Reste steht und

$R^2$ für Wasserstoff oder für gegebenenfalls substituierte Alkyl-, Alkenyl-, Alkinyl- und Cycloalkyl-Reste steht.

Sie werden erhalten, indem man 4-Hydroxyimidazole mit N,N-Dimethylcarbamidsäurehalogeniden der Formel

$$(CH_3)_2N\text{–}CO\text{–}Hal \qquad (III)$$

umsetzt.

Die neuen N,N-Dimethyl-O-imidazol-4-yl-carbaminsäureester der Formel (I) zeichnen sich durch hohe Wirksamkeit als Schädlingsbekämpfungsmittel aus.

**EP 0 075 144 A1**

0075144

BAYER AKTIENGESELLSCHAFT 5090 Leverkusen, Bayerwerk
Zentralbereich Rt/Fr
Patente, Marken und Lizenzen Ia

11. 09. 81

N,N-Dimethyl-O-imidazol-4-yl-carbaminsäureester, Verfahren zu ihrer Herstellung und ihre Verwendung als Schädlingsbekämpfungsmittel

Die Erfindung betrifft neue N,N-Dimethyl-O-imidazol-4-yl-carbaminsäureester, ein Verfahren zu ihrer Herstellung und ihre Verwendung als Schädlingsbekämpfungsmittel, insbesondere als Insektizide.

Es ist bekannt, daß bestimmte Carbaminsäureester, wie z.B. N,N-Dimethyl-O-[1-methyl-imidazol-5-yl]-carbaminsäureester, N,N-Dimethyl[1-dimethylamino-4-methyl-2-methylthio-imidazol-5-yl]-carbaminsäureester und N,N-Dimethyl[1-isopropyl-imidazol-5-yl]-carbaminsäureester pestizid wirksam sind (vergleiche DE-OS 2 611 57o).

Die Wirkung dieser Verbindungen ist jedoch, insbesondere bei niedrigen Wirkstoffkonzentrationen und Aufwandmengen, nicht immer ganz zufriedenstellend.

Es wurden neue N,N-Dimethyl-O-imidazol-4-yl-carbaminsäureester der Formel (I) gefunden

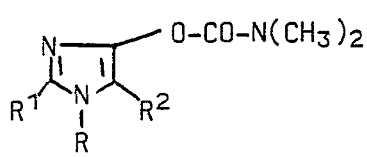

(I)

in welcher

R für gegebenenfalls substituierte Alkyl-, Alkenyl-, Alkinyl- und Cycloalkylreste steht,

$R^1$ für Wasserstoff oder für gegebenenfalls substituierte Alkyl-, Alkenyl-, Alkinyl- und Cycloalkyl-reste steht, und

$R^2$ für Wasserstoff oder für gegebenenfalls substituierte Alkyl-, Alkenyl-, Alkinyl- und Cycloalkyl-restesteht.

Man erhält die neuen N,N-Dimethyl-O-imidazol-4-yl-carbaminsäureester der Formel (I), wenn man 4-Hydroxyimidazole der Formel

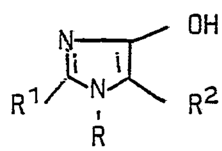

(II)

in welcher

R, $R^1$ und $R^2$ die oben angegebenen Bedeutungen haben,

gegebenenfalls in Gegenwart eines Verdünnungsmittels und gegebenenfalls in Gegenwart von Säurebindern mit N,N-Dimethylcarbamidsäurehalogeniden der Formel

$(CH_3)_2N-CO-Hal$ (III)

in welcher

Hal für Fluor, Chlor oder Brom steht,

umsetzt.

Die neuen N,N-Dimethyl-O-imidazol-4-yl-carbaminsäureester der Formel (I) zeichnen sich durch hohe Wirksamkeit als Schädlingsbekämpfungsmittel, insbesondere durch hohe insektizide Wirksamkeit aus.

Ueberraschenderweise zeigen die erfindungsgemäßen Verbindungen der Formel (I) erheblich höhere insektizide Wirkung als die bereits erwähnten Verbindungen ähnlicher Konstitution und gleicher Wirkungsrichtung. Besonders hervorzuheben ist ihre systemische Wirkung auf Insekten.

Die erfindungsgemäßen N,N-Dimethyl-O-imidazol-4-yl-carbaminsäureester sind durch die Formel (I) allgemein definiert. In dieser Formel steht R vorzugsweise für gegebenenfalls substituierte geradkettige oder verzweigte Alkyl-reste mit 1 bis 6 Kohlenstoffatomen, Alkenyl- und Alkinyl-reste mit 2 bis 5 Kohlenstoffatomen und Cycloalkyl-reste mit 3 bis 7 Kohlenstoffatomen.

$R^1$ und $R^2$ stehen vorzugsweise und unabhängig voneinander für Wasserstoff oder für gegebenenfalls substituierte geradkettige oder verzweigte Alkyl-reste mit 1 bis 6 Kohlenstoffatomen, Alkenyl- und Alkinylreste mit 2 bis 5 Kohlenstoffatomen, und Cycloalkyl-reste mit 3 bis 7 Kohlenstoffatomen.

Als Substituenten kommen vorzugsweise infrage: Halogen, wie Fluor, Chlor oder Brom, Alkyl-reste mit 1 bis 4 Kohlenstoffatomen, Alkylthio-, Alkylsulfinyl- und Alkylsulfonylreste mit jeweils 1 bis 4 Kohlenstoffatomen im Alkylteil.

Ganz besonders bevorzugt sind diejenigen N,N-Dimethyl-O-imidazol-4-yl-carbaminsäureester der Formel (I), in denen

R für Methyl, Ethyl, n-Propyl, iso-Propyl, n-Butyl, iso-Butyl, sec-Butyl, tert-Butyl, 2-Methyl-butyl, Cyclopropyl, Cyclobutyl, Cyclopentyl Cyclohexyl, Allyl und Propargyl steht;

$R^1$ und $R^2$ unabhängig voneinander für Wasserstoff, Methyl, Ethyl, n-Propyl, iso-Propyl, n-Butyl, iso-Butyl, sec-Butyl, tert-Butyl, 2-Methylbutyl, Cyclopropyl, Cyclobutyl, Cyclohexyl, Cyclopentyl, Alkyl, Propargyl, Methylthiomethyl, Ethylthiomethyl, Methylthioethyl,

n-Propylthiomethyl, iso-Propylthiomethyl, Methylsulfinylmethyl, Ethylsulfinylethyl, Methylsulfinylethyl, Methylsulfonylmethyl, Ethylsulfonylmethyl und Methylsulfonylethyl stehen.

Verwendet man als Ausgangsstoffe beispielsweise 2-tert-Butyl-1-ethyl-4-hydroxy-imidazol und N,N-Dimethylcarbamidsäurechlorid als Ausgangsstoffe, so kann der Reaktionsablauf durch das folgende Formelschema wiedergegeben werden:

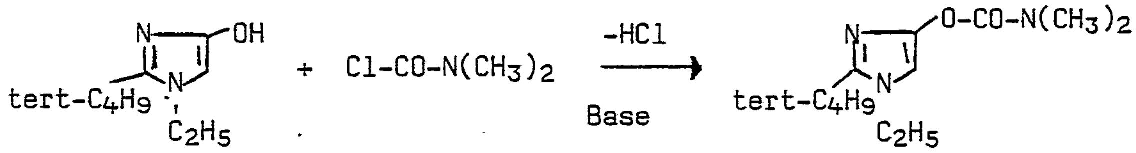


Die als Ausgangsstoffe zu verwendenden 4-Hydroxyimidazole sind durch die Formel (II) allgemein definiert. In dieser Formel steht $R^2$ vorzugsweise für diejenigen Reste, die bereits im Zusammenhang mit der Beschreibung der erfindungsgemäßen Stoffe der Formel (I) vorzugsweise für diese Substituenten genannt wurden.

Als Beispiele seien genannt:

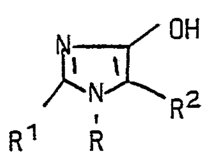
(II)

| R | $R^1$ | $R^2$ |
|---|---|---|
| $C_3H_7$-iso | $C_2H_5$ | H |
| $C_3H_7$-iso | $CH_3$ | H |
| $C_3H_7$-iso | $C_3H_7$-n | H |

| R | $R^1$ | $R^2$ |
|---|---|---|
| $C_3H_7$-iso | $CH_2$-$SCH_3$ | H |
| $CH_3$ | $C_3H_7$-iso | H |
| $C_3H_7$-iso | $CH_3$ | $CH_3$ |
| $CH_3$ | $CH_3$ | $C_3H_7$-iso |
| $C_3H_7$-iso | $CH_3$ | $C_2H_5$ |
| $CH_3$ | $C_2H_5$ | $C_3H_7$-n |
| 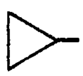 | $CH_3$ | H |
| $C_4H_9$-tert | $CH_3$ | H |
| $C_3H_7$-iso | $CH_2$-SO-$CH_3$ | H |
| $C_3H_7$-iso | $CH_2$-$SO_2$-$CH_3$ | H |
| $C_3H_7$-iso | $CH_2$-$SCH_3$ | $CH_3$ |
| $C_3H_7$-iso | 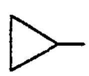 | H |
| 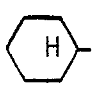 | $CH_3$ | H |
| $CH_3$ | $C_3H_7$-iso | $C_3H_7$-iso |
| $C_3H_7$-iso | 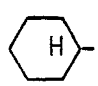 | H |

| R | $R^1$ | $R^2$ |
|---|---|---|
| $CH_3$ | $C_4H_9$-tert | H |
| $C_4H_9$-tert | $CH_3$ | $CH_3$ |
| $H_5C_2-C(CH_3)_2-$ | $CH_3$ | H |
| $C_3H_7$-iso | $C_4H_9$-tert | H |
| $C_2H_5$ | $CH_3$ | H |

Die 4-Hydroxy-imidazole der Formel (II) sind teilweise bekannt (vergleiche Chem.Ber. 89, 1363-1373 (1956). Man erhält sie beispielseise durch Umsetzung von Orthoestern mit substituierten α-Aminosäureamiden. Die Herstellung der substituierten α-Aminosäureamide erfolgt z.B. auf bekannte Weise aus Chloracetamid durch Umsetzung mit entsprechendem Amin [vergleiche Chem.Ber. 89, 1363-1373 (1956)] .

Die weiterhin für das erfindungsgemäße Verfahren als Ausgangsstoffe zu verwendenden N,N-Dimethylcarbamidsäurehalogenide sind durch die Formel (III) allgemein definiert. In dieser Formel steht Hal vorzugsweise für Fluor, Chlor oder Brom.

Als Beispiel seien genannt: N,N-Dimethylcarbamidsäure-fluorid, -chlorid und -bromid.

Die N,N-Dimethylcarbamidsäurehalogenide sind bekannt oder lassen sich nach allgemein üblichen und bekannten Verfahren herstellen.

Als Verdünnungsmittel kommen für die erfindungsgemäße Umsetzung vorzugsweise alle inerten organischen Lösungsmittel infrage. Hierzu gehören insbesondere aliphatische und aromatische, gegebenenfalls chlorierte Kohlenwasserstoffe, wie Benzin, Benzol, Toluol, Xylol, Methylenchlorid, Chloroform, Tetrachlorkohlenstoff, Chlorbenzol und o-Dichlorbenzol, Ether wie Diethylether und Dibutylether, Tetrahydrofuran und Dioxan, Ketone wie Aceton, Methylethyl- und Methylisopropyl- und Methylisobutylketon sowie Nitrile, wie Acetonitril und Propionitril.

Das erfindungsgemäße Verfahren wird in Gegenwart von Säurebindern vorgenommen. Man kann alle üblicherweise verwendbaren anorganischen und organischen Säurebinder zugeben. Hierzu gehören vorzugsweise Alkalicarbonate, wie beispielsweise Natriumcarbonat, Kaliumcarbonat und Natriumhydrogencarbonat, ferner niedere tertiäre Alkylamine, Cycloalkylamine oder Arylalkylamine, wie beispielsweise Triethylamin, N,N-Dimethyl-benzylamin, weiterhin Pyridin sowie 1,4-Diazabicyclo(2,2,2)-octan und 1,5-Diazabicyclo-(4,3,o)-non-5-en.

Die Reaktionstemperatur kann innerhalb eines größeren Bereichs variiert werden. Im allgemeinen arbeitet man zwischen 0 und 12o°C, vorzugsweise zwischen lo und 9o°C.

Das erfindungsgemäße Verfahren wird im allgemeinen bei Normaldruck durchgeführt.

Zur Durchführung des erfindungsgemäßen Verfahrens setzt man auf 1 Mol 4-Hydroxyimidazol der Formel (II) gewöhnlich zwischen 1 bis 1,5 Mol, vorzugsweise zwischen 1 und 1,2 Mol N,N-Dimethylcarbamidsäurehalogenid der Formel (III) ein

Die Umsetzung wird vorzugsweise unter Verwendung eines der oben genannten Säurebindern in einem der oben angegebenen Verdünnungsmittel durchgeführt. Das Reaktionsgemisch wird mehrere Stunden bei der erforderlichen Temperatur gerührt. Dann wird das Lösungsmittel im Vakuum abdestilliert. Man erhält so die Produkte in öliger oder kristalliner Form. Zur Charakterisierung dient der Schmelzpunkt oder der Brechungsindex.

Die erfindungsgemäßen N,N-Dimethyl-O-imidazol-4-yl-carbaminsäureester zeichnen sich durch hervorragende insektizide Wirkung aus.

Die Wirkstoffe eignen sich bei guter Pflanzenverträglichkeit und günstiger Warmblütertoxizität zur Bekämpfung von tierischen Schädlingen, insbesondere Insekten, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp.,Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp. Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp.,

Trogoderma spp., Anthrenus spp., Attagenus spp., Lyctus spp., Meligethes aeneus, Ptinus spp., Niptus hololeucus Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B.. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopsis, Ceratophyllus spp.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff

und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo - und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

0075144

Herstellungsbeispiele

Beispiel 1:

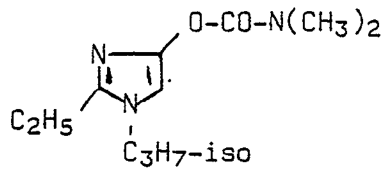

Eine Mischung aus 15,4 g (o,1 Mol) 1-Isopropyl-2-ethyl-4-hydroxy-imidazol, 2o,7 g (o,15 Mol) Kaliumcarbonat, 12 g (o,11 Mol) Dimethylcarbamidsäurechlorid und 2oo ml Acetonitril wird 18 Stunden bei 7o°C gerührt. Nach dem Abkühlen saugt man vom anorganischen Salz ab und dampft das Filtrat im Vakuum ein. Der Rückstand wird bei 5o°C im Hochvakuum andestilliert. Man erhält so 1o,8 g (48% der Theorie) N,N-Dimethyl-O-[1-isopropyl-2-ethyl-imidazol(4)yl]-carbamidsäureester in Form eines rotbraunen Oeles mit dem Brechungsindex $n_D^{24}$: 1,495o.

In analoger Weise können die folgenden Verbindungen der Formel

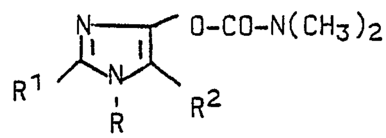

(I)

hergestellt werden (Tabelle 1):

Tabelle 1:

| Beispiel Nr. | R | $R^1$ | $R^2$ | Ausbeute [% der Theorie] | Brechungsindex; Schmelzpunkt [°C] |
|---|---|---|---|---|---|
| 2 | $C_3H_7$-iso | $CH_3$ | H | 88 | |
| 3 | $C_3H_7$-iso | $C_3H_7$-n | H | 63 | $n_D^{21}$:1,4999 |
| 4 | $C_3H_7$-iso | $CH_2$-$SCH_3$ | H | 61 | $n_D^{23}$:1,5328 |
| 5 | $CH_3$ | $C_3H_7$-iso | H | | |
| 6 | $C_3H_7$-iso | $CH_3$ | $CH_3$ | | |
| 7 | $CH_3$ | $CH_3$ | $C_3H_7$-iso | | |
| 8 | $C_3H_7$-iso | $CH_3$ | $C_2H_5$ | | |
| 9 | $CH_3$ | $C_2H_5$ | $C_3H_7$-n | | |
| 1o | 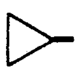 | $CH_3$ | H | | |
| 11 | $C_4H_9$-tert | $CH_3$ | H | | |
| 12 | $C_3H_7$-iso | $CH_2$-SO-$CH_3$ | H | | |
| 13 | $C_3H_7$-iso | $CH_2$-$SO_2$-$CH_3$ | H | | |
| 14 | $C_3H_7$-iso | $CH_2$-$SCH_3$ | $CH_3$ | | |
| 15 | $C_3H_7$-iso | 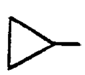 | H | | |
| 16 | 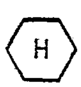 | $CH_3$ | H | | |

| Bei-<br>spiel<br>Nr. | R | $R^1$ | $R^2$ | Ausbeute<br>[% der<br>Theorie] | Brechungsindex;<br>Schmelzpunkt<br>[°C] |
|---|---|---|---|---|---|
| 17 | $CH_3$ | $C_3H_7$-iso | $C_3H_7$-iso | | |
| 18 | $C_3H_7$-iso | 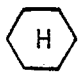 | H | | |
| 19 | $CH_3$ | $C_4H_9$-tert | H | | |
| 20 | $C_4H_9$-tert | $CH_3$ | $CH_3$ | | |
| 21 | $CH_3$<br>$CH_3$-$CH_2$-C-<br>$CH_3$ | $CH_3$ | H | | |
| 22 | $C_3H_7$-iso | $C_4H_9$-tert | H | | |
| 23 | $C_2H_5$ | $CH_3$ | H | | |

Herstellung der Ausgangsprodukte

Beispiel a: 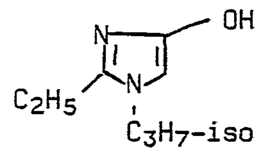

Eine Mischung aus 8o g (o,45 Mol) Orthopropionsäure-triethylester, 46,4 g (o,4 Mol) α-Isopropylaminoacetamid und 2 ml Eisessig wird in einem Heizbad von 14o-15o°C erwärmt und dabei das entstehende Ethanol abdestilliert. Nach Ende der Reaktion kühlt man auf 6o°C ab, gibt loo ml Eisessig zu und kühlt dann weiter auf 0-5°C ab. Nach Kristallisation saugt man das Reaktionsprodukt ab. Man erhält so 48,9 g (8o% der Theorie) 1-Isopropyl-2-ethyl-4-hydroxyimidazol in Form eines rosa Pulvers mit dem Schmelzpunkt 132°C.

Analog Beispiel a können die folgenden Verbindungen der Formel

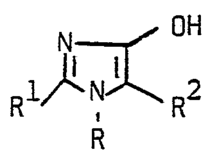

(II)

hergestellt werden (Tabelle 2):

Tabelle 2:

| Beispiel Nr. | R | $R^1$ | $R^2$ | Ausbeute [% der Theorie] | Schmelzpunkt [°C] |
|---|---|---|---|---|---|
| b | $C_3H_7$-iso | $CH_3$ | H | 86 | 113 |
| c | $C_3H_7$-iso | $C_3H_7$-n | H | 57 | 120 |
| d | $C_3H_7$-iso | $CH_2$-$SCH_3$ | H | 65 | 117 |
| e | $CH_3$ | $C_3H_7$-iso | H | | |
| f | $C_3H_7$-iso | $CH_3$ | $CH_3$ | | |
| g | $CH_3$ | $CH_3$ | $C_3H_7$-iso | | |
| h | $C_3H_7$-iso | $CH_3$ | $C_2H_5$ | | |
| i | $CH_3$ | $C_2H_5$ | $C_3H_7$-n | | |
| j | 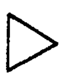 | $CH_3$ | H | | |
| k | $C_4H_9$-tert | $CH_3$ | H | | |
| l | $C_3H_7$-iso | $CH_2$-SO-$CH_3$ | H | | |
| m | $C_3H_7$-iso | $CH_2$-$SO_2$-$CH_3$ | H | | |
| n | $C_3H_7$-iso | $CH_2$-$SCH_3$ | $CH_3$ | | |
| o | $C_3H_7$-iso | 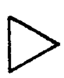 | H | | |

| Beispiel Nr. | R | $R^1$ | $R^2$ | Ausbeute [% der Theorie] | Schmelzpunkt [°C] |
|---|---|---|---|---|---|
| p | 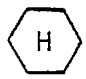 | $CH_3$ | H | | |
| q | $CH_3$ | $C_3H_7$-iso | $C_3H_7$-iso | | |
| r | $C_3H_7$-iso | 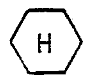 | H | | |
| s | $CH_3$ | $C_4H_9$-tert | H | | |
| t | $C_4H_9$-tert | $CH_3$ | $CH_3$ | | |
| u | 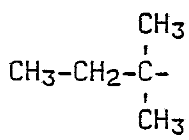 | $CH_3$ | H | | |
| v | $C_3H_7$-iso | $C_4H_9$-tert | H | | |
| w | $C_2H_5$ | $CH_3$ | H | | |

0075144

Beispiel A

Phaedon-Larven-Test

Lösungsmittel: 3 Gewichtsteile Dimethylformamid
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100 %, daß alle Käfer-Larven abgetötet wurden; 0 % bedeutet, daß keine Käfer-Larven abgetötet wurden.

Bei diesem Test zeigen z.B. die folgenden Verbindungen der Herstellungsbeispiele überlegene Wirksamkeit gegenüber dem Stand der Technik: 1, 2, 3, 4

Beispiel B

Doralis-Test (systemische Wirkung)

Lösungsmittel: 3 Gewichtsteile Dimethylformamid
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Mit je 20 ml Wirkstoffzubereitung der gewünschten Konzentration werden Bohnenpflanzen (Vicia faba), die stark von der schwarzen Bohnenlaus (Doralis fabae) befallen sind, angegossen, so daß die Wirkstoffzubereitung in den Boden eindringt, ohne den Sproß zu benetzen. Der Wirkstoff wird von den Wurzeln aufgenommen und in den Sproß weitergeleitet.

Nach der gewünschten Zeit wird die Abtötung in % bestimmt. Dabei bedeutet 100 %, daß alle Blattläuse abgetötet wurden; 0 % bedeutet, daß keine Blattläuse abgetötet wurden.

Bei diesem Test zeigen z.B. die folgenden Verbindungen der Herstellungsbeispiele überlegene Wirksamkeit gegenüber dem Stand der Technik: 1, 2, 3, 4

Beispiel C

Grenzkonzentrations-Test / Wurzelsystemische Wirkung

Testinsekt: Myzus Persicae
Lösungsmittel: 3 Gewichtsteile Aceton
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (=mg/l) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle Testtiere abgetötet sind und 0 %, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik: 1, 2, 3, 4

Beispiel D

Grenzkonzentrations-Test / Wurzelsystemische Wirkung

Testinsekt: Phaedon cochleariae-Larven

Lösungsmittel: 3 Gewichtsteile Aceton

Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (=mg/l) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle Testtiere abgetötet sind und 0 %, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik: 1, 2, 3, 4

Patentansprüche

1. N,N-Dimethyl-O-imidazol-4-yl-carbaminsäureester der Formel (I)

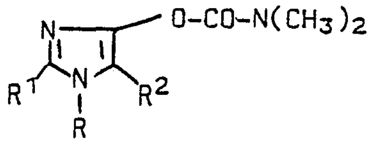

(I)

in welcher

R für gegebenenfalls substituierte Alkyl-, Alkenyl-, Alkinyl- und Cycloalkylreste steht,

$R^1$ für Wasserstoff oder für gegebenenfalls substituierte Alkyl-, Alkenyl-, Alkinyl- und Cycloalkyl-reste steht, und

$R^2$ für Wasserstoff oder für gegebenenfalls substituierte Alkyl-, Alkenyl-, Alkinyl- und Cycloalkyl-restesteht.

2. Verfahren zur Herstellung der N,N-Dimethyl-O-imidazol-4-yl-carbaminsäureester der Formel (I)

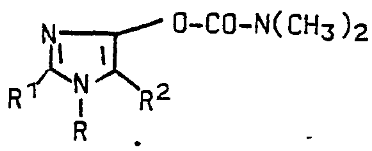

(I)

in welcher

R für gegebenenfalls substituierte Alkyl-, Alkenyl-, Alkinyl- und Cycloalkylreste steht,

$R^1$ für Wasserstoff oder für gegebenenfalls substituierte Alkyl-, Alkenyl-, Alkinyl- und Cycloalkyl-reste steht, und

$R^2$ für Wasserstoff oder für gegebenenfalls substituierte Alkyl-, Alkenyl-, Alkinyl- und Cycloalkyl-restesteht,

0075144

dadurch gekennzeichnet, daß man 4-Hydroxyimidazole der Formel (II)

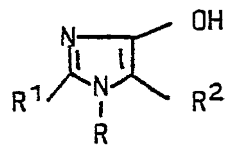

(II)

in welcher

R, $R^1$ und $R^2$ die oben angegebenen Bedeutungen haben,

gegebenenfalls in Gegenwart eines Verdünnungsmittels und gegebenenfalls in Gegenwart von Säurebindern mit N,N-Dimethylcarbamidsäurehalogeniden der Formel

$$(CH_3)_2N\text{-}CO\text{-}Hal \qquad (III)$$

in welcher

Hal für Fluor, Chlor oder Brom steht,

umsetzt.

3. N,N-Dimethyl-O-imidazol-4-yl-carbaminsäureester der Formel (I) gemäß Anspruch 1, in welcher
R für gegebenenfalls substituierte geradkettige oder verzweigte Alkyl-reste mit 1 bis 6 Kohlenstoffatomen, Alkenyl- und Alkinyl-reste mit 2 bis 5 Kohlenstoffatomen und Cycloalkyl-reste mit 3 bis 7 Kohlenstoffatomen steht,

$R^1$ und $R^2$ unabhängig voneinander für Wasserstoff oder für gegebenenfalls substituierte geradkettige oder verzweigte Alkyl-reste mit 1 bis 6 Kohlenstoffatomen,

Alkenyl- und Alkinylreste mit 2 bis 5 Kohlenstoffatomen, und Cycloalkyl-reste mit 3 bis 7 Kohlenstoffatomen stehen,

wobei als Substituenten infrage kommen: Fluor, Chlor oder Brom, Alkyl-reste mit 1 bis 4 Kohlenstoffatomen, Alkylthio-, Alkylsulfinyl- und Alkylsulfonyl-reste mit jeweils 1 bis 4 Kohlenstoffatomen im Alkylteil.

4. N,N-Dimethyl-O-imidazol-4-yl-carbaminsäureester gemäß Anspruch 1, der Formel (I), in welcher
R für Methyl, Ethyl, n-Propyl oder iso-Propyl steht;
$R^1$ und $R^2$ unabhängig voneinander für Wasserstoff, Methyl, Ethyl, n-Propyl, iso-Propyl, Methylthiomethyl, Ethylthiomethyl oder Methylthioethyl steht.

5. Schädlingsbekämpfungsmittel, gekennzeichnet durch einen Gehalt an mindestens einem N,N-Dimethyl-O-imidazol-4-yl-carbaminsäureester der Formel (I).

6. Verwendung von N,N-Dimethyl-O-imidazol-4-yl-carbaminsäureester der Formel (I) zur Bekämpfung von Schädlingen.

7. Verfahren zur Bekämpfung von Schädlingen, dadurch gekennzeichnet, daß man N,N-Dimethyl-O-imidazol-4-yl-carbaminsäureester der Formel (I) auf Schädlinge und/oder ihren Lebensraum einwirken läßt.

8. Verfahren zur Herstellung von Schädlingsbekämpfungsmitteln, dadurch gekennzeichnet, daß man N,N-Dimethyl-O-imidazol-4-yl-carbaminsäureester der Formel (I) mit Streckmitteln und/oder oberflächenaktiven Mitteln vermischt.

Europäisches Patentamt

# EUROPÄISCHER RECHERCHENBERICHT

0075144 Nummer der Anmeldung

EP 82 10 7998

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
| D,A | --- DE-A-2 611 770 (BAYER) ----- | | C 07 D 233/70 A 01 N 43/50 |
| | | | RECHERCHIERTE SACHGEBIETE (Int. Cl. 3) |
| | | | C 07 D 233/00 A 01 N 43/00 |

Der vorliegende Recherchenbericht wurde für alle Patentansprüche erstellt.

| Recherchenort | Abschlußdatum der Recherche | Prüfer |
|---|---|---|
| DEN HAAG | 20-12-1982 | DE BUYSER I.A.F. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1503. 03.82